# EUROPEAN PATENT APPLICATION

(11) **EP 2 517 632 A2**
(43) Date of publication of application: **31.10.2012**
(21) Application number: 12275052.4
(22) Date of filing: 27.04.2012
(51) Int. Cl.: A61B 17/00, A61F 2/06

(54) **Fenestration sealing device**

(30) Priority: 28.04.2011 US 201161480084 P
(71) Applicant: Greenberg, Roy K., Bratenahl, OH 44108 (US)
(72) Inventor: Greenberg, Roy K., Bratenahl, OH 44108 (US)
(74) Representative: Jehan, Robert

(57) **Abstract**

A sealing device (10) is configured to seal a fenestration (30) with a stent graft (26). In one example, the sealing device (10) has a biasing member (18) that is used to retain a sealing member (12) adjacent to the fenestration (30) to prevent blood from escaping from the stent graft (26). The biasing member (18) is configured to pull the sealing member (12) against the fenestration (30) to ensure a proper seal.

## Description

The present invention relates to a sealing device for sealing a fenestration within a graft.

Fenestrated devices, such as grafts and stent grafts for treating aortic aneurysms, such as abdominal aortic aneurysms and thoracic aortic aneurysms, are known for use in treating conditions of body vessels where the body vessel has one or more branch vessels so as not to obstruct the branch vessel. Because of the vagaries of individual patient anatomy, precise placement of the fenestration to align with the orifice of the branch vessel may present a challenge. For example, the anatomy of the patient may have changed from the time of imaging the location of the branching vessel(s) to the time of placement of the device. In such instances, it may be desirable to implant a device that has two or more fenestrations in the graft that may be selected from for alignment with the branch vessel. Once the appropriate fenestration is aligned with the vessel, the other fenestrations would need to be sealed.

Further, fenestrations in such devices also may be used for catheter access or for access by other devices. Once the required access is no longer needed, the fenestration would need to be sealed to avoid leakage. Some devices are used to seal a fenestration that is not needed or no longer required for access. For example, a valve or flap arrangement may be used to seal the fenestration from the inner portion of the stent graft. These types of sealing devices typically require that the device be inserted along with the bifurcated stent graft and placed in the expanded configurations. In order to access the fenestration, the catheter must be maneuvered between the valve or flap and the inner wall of the stent graft to gain access to the area outside of the stent graft.

The present invention seeks to provide improved sealing of grafts.

An aspect of the present invention provides a sealing device as specified in claim 1.

In one embodiment, a sealing device for sealing a fenestration in a wall of a prosthesis such as a graft or stent-graft is provided. The prosthesis has an interior surface and an exterior surface. The sealing device includes a sealing member that has an exterior face and an interior face, and an outer diameter that is greater than the diameter of the fenestration. The sealing device also includes a biasing member that extends from the exterior face of the sealing member. The biasing member extends through the fenestration, for example of a tubular graft, and applies a force against the exterior surface of the graft to cause the sealing member to abut the inside surface of the graft around the fenestration. The biasing member may include at least two biasing fingers coupled to the sealing member, where the at least two biasing fingers extend away from the exterior face of the sealing member and are configured to extend through the fenestration and apply a force against exterior portion of the graft to cause the sealing member to sealing abut the fenestration.

Another aspect of the present invention provides a sealing device for sealing a fenestration within a graft, the sealing device comprising a sealing member having an exterior face and an interior face, where the outer diameter of the sealing member is greater than the outer diameter of the fenestration; and at least two biasing fingers coupled to the sealing member, where the at least two biasing fingers extend away from the exterior face of the sealing member and are configured to extend through the fenestration and apply a force against an exterior portion of the graft to cause the sealing member to abut the fenestration.

Embodiments of the present invention are described below by way of example only, with reference to the accompanying drawings, in which:
Figure 1 shows a first embodiment of the sealing device in a natural state.
Figure 2 shows the embodiment of Figure 1 engaged with the stent graft to seal the fenestration.
Figure 3 shows the embodiment of Figure 1 within the delivery system.
Figure 4 shows another embodiment of the sealing device in a natural state.
Figure 5 shows the embodiment of Figure 4 engaged with the stent graft to seal the fenestration.
Figure 6 shows the embodiment of Figure 4 within the delivery system.
Figure 7 shows another embodiment of the sealing device in a nature state.
Figure 8 shows the embodiment of Figure 7 engaged with the stent graft to seal the fenestration.
Figure 9 shows the embodiment of Figure 7 within the delivery system.
Figure 10 shows a top view of yet another embodiment of the sealing device in a natural state.
Figure 11 shows a side view of the embodiment of Figure 10 in the natural state.
Figure 12 shows the embodiment of Figure 10 engaged with the stent graft to seal the fenestration.
Figure 13 shows the embodiment of Figure 10 within the delivery system.
Figure 14 shows a side view of yet another embodiment of the sealing device in a natural state.
Figure 15 shows the embodiment of Figure 14 within the delivery system.
Figure 16 shows a side view of the embodiment of Figure 14 in the expanded configuration.
Figure 17 shows the delivery system prior to deploying the sealing device.
Figure 18 shows the delivery system of Figure 17 with a portion of the sealing device deployed.
Figure 19 shows the delivery system of claim 17 with the sealing device completely deployed and secured to the stent graft.

### DETAILED DESCRIPTION OF THE DRAWINGS AND THE PRESENTLY PREFERRED EMBODIMENTS

The embodiments below are described with reference to the drawings in which like elements are referred to by like numerals. The relationship and functioning of the various elements are better understood by the following detailed description. However, the embodiments as described below are by way of example only, and the embodiments are not limited to the embodiments illustrated in the drawings.

The present embodiments relate to a sealing device used to seal a fenestration formed within a stent graft where the sealing device includes a sealing member to abut the fenestration and a biasing member to maintain the sealing member in a position adjacent to the fenestration to prevent blood from escaping through the fenestration. Methods of delivery and placement of the sealing device to seal a fenestration are also shown and described herein.

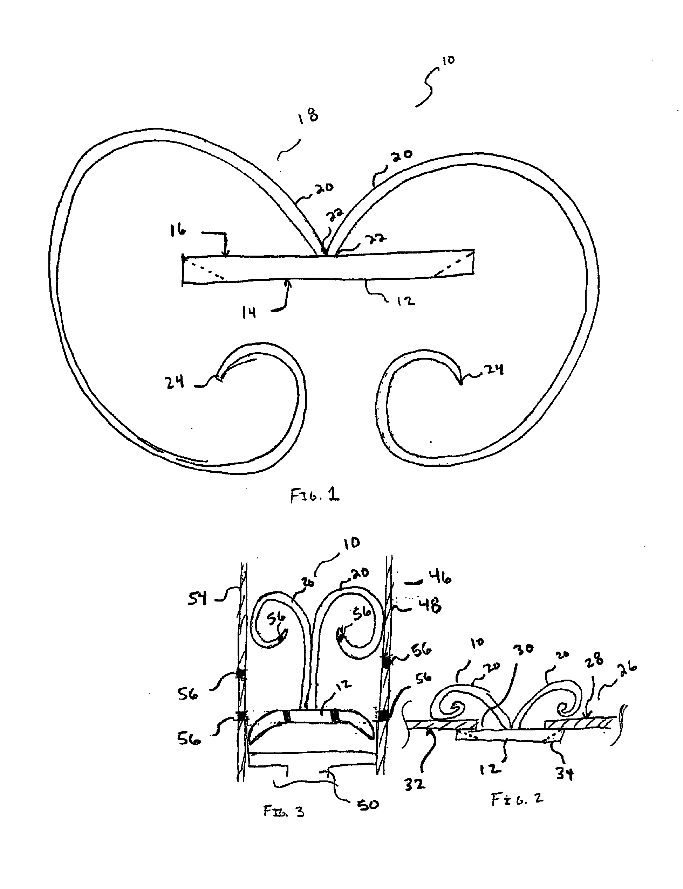

In one embodiment, shown in Figure 1, the sealing device 10 includes a sealing member 12 having an exterior face 14 and an interior face 16. The sealing member 12 may be made out of graft material that is commonly used and available, such as polytetrafluoroethylene (ePTFE), Thoralon^{™}, Dacron, polyamide or any other suitable biocompatible graft material. A naturally occurring biomaterial, such as collagen, may also be suitable, particularly a specially derived collagen material known as an extracellular matrix (ECM) material, such as submucosa, renal capsule membrane, dermal collagen, dura mater, pericardium, fascia lata, serosa, peritoneum or basement membrane layers, including liver basement membrane. Suitable submucosa materials for these purposes include, for instance, intestinal submucosa, including small intestinal submucosa (SIS), stomach submucosa, urinary bladder submucosa, and uterine submucosa.

In this embodiment, the sealing member 12 is circular in shape and has a substantially constant thickness; however, it is appreciated that the shape and thickness may be varied depending on the particular application that it is being used for. Attached to the sealing member 12 is a biasing member 18, as shown in Figure 1. The biasing member 18 may be connected to the sealing member 12 in a plurality of ways, including being anchored, stitched, adhered, or using mechanical devices such as clamps, clips, snap-fit arrangements, and the like. Additionally, other mechanisms known now or that may become known to one of skill in the art may be used to connect the biasing member 18 to the sealing member 12. In one embodiment, the biasing member 18 may be attached to a frame or be formed as part of the reinforcing member.

As shown in Figure 1, the biasing member 18 comprises two biasing fingers 20, each having a first end 22 and a second end 24. As shown in Figure 1, the first end 22 of each biasing finger 20 is secured to the sealing member 12 in a manner identified above. The second end 24 of the biasing finger 20 may have a curved or helical shape to help center the sealing device with respect to a fenestration. The curved shape may also help allow the biasing fingers 20 to rotate with respect to the outer wall 28 of the stent graft 26 to pull the sealing member 12 against the fenestration 30. The types of material from which the biasing fingers 20 may be manufactured include a shape memory alloy comprised of Nickel and Titanium, which is commonly referred to as Nitinol, and Teritary alloys (or "Nitinol"). The biasing fingers 20 may be made from other metals and alloys that are biased, such that they may be restrained prior to deployment, but are inclined to return to their relaxed, expanded configuration upon deployment. Solely by way of example, the biasing fingers 20 may comprise other materials such as stainless steel, cobalt-chrome alloys, amorphous metals, tantalum, platinum, gold and titanium, or may be made from non-metallic materials, such as thermoplastics and other polymers.

As further shown in Figure 1, when in a natural state, the biasing fingers 20 of the sealing device originate from the exterior face 14 and curl around the sides of the sealing member 12. The preformed curve of the biasing fingers 20 in the natural state as shown in Figure 2 allow the sealing device 10 to seal the fenestration 30 and prevent fluids, such as blood, from escaping from the stent graft. In order to sealingly engage the fenestration 30, the biasing fingers supply sufficient force against an outer wall 28 of the stent graft 26 to effectively pull the sealing member 12 against the fenestration 30. The amount of force required is application and material dependent, for example the thickness or rigidity of the material, and is preferably in excess of the displacement forces. Displacement forces may include hemodynamic forces, physical forces, respiratory forces and any other forces that might act upon the sealing member, To supply such a force, the biasing fingers 20 typically have an outer diameter ranging from about 0.3mm to about 1.5mm, and may comprise a length of about 2.0 mm to about 30.0mm. To maximize the pulling force, the outer diameter of each biasing finger 20 may vary along its length and the length of the each of the biasing fingers 20 for a particular application may vary. In addition, the number of biasing fingers 20 may vary depending on a particular application.

Figure 2 illustrates a side view of the sealing device 10 secured to the outer wall 28 of the stent graft 26 to seal the fenestration 30. In this embodiment, the biasing fingers 20 are biased against the outer wall 28 of the stent graft 26 such that the sealing member 12 is pulled flush against the fenestration 30, thereby sealing the fenestration 30 to prevent blood from escaping therethrough. The curved shape of the second ends 24 of the biasing fingers 20 encourage the fingers 20 to curl, thereby increasing the biasing force and further securing the sealing member 12 against the inner wall 32 of the stent graft 26 surrounding the fenestration 30.

The exterior face 14 of the sealing member 12 has an area greater than the area formed by the fenestration 30 to effectively seal the opening. The amount of overlap between the sealing member 12 on the inner wall 32 of the stent graft 26 is determined based at least in part on the amount of pressure exerted by the blood flowing through the stent graft 26 and the biasing force exerted by the biasing member 18. In most cases, the amount of overlap may be between about 0.5 and about 10.0 mm. In one embodiment, the sealing device may comprise a diameter of 10% or more greater than the diameter of the fenestrations. In another embodiment, the sealing device may be asymmetrically shaped or oppose the convexity of the aortic graft to achieve a better seal and fixation over the fenestration. To prevent the sealing member 12 from moving, rotating, or sliding relative to the stent graft 26 after insertion, the exterior face 14 of the sealing member may have a rough surface along its outer portion so as to enhance the frictional force as that portion comes into contact with the inner wall 32 of the stent graft 26.

In addition, the outer edges 34 of the sealing member 12 may be tapered, as shown in dashed lines on Figures 1 and 2. The tapering of the edges of the sealing member 12 will reduce the disruption of the blood flow through the stent graft 26. The amount of taper may vary between about 0.1 and about 45.0 degrees to encourage laminar blood flow and to provide a substantially imperceptible gradient between the sealing device and the wall of the stent graft or a hole in the aorta. It can also be appreciated that the entire perimeter of the sealing device 12 may not be tapered but only the portions which are facing the direction of the blood flow.

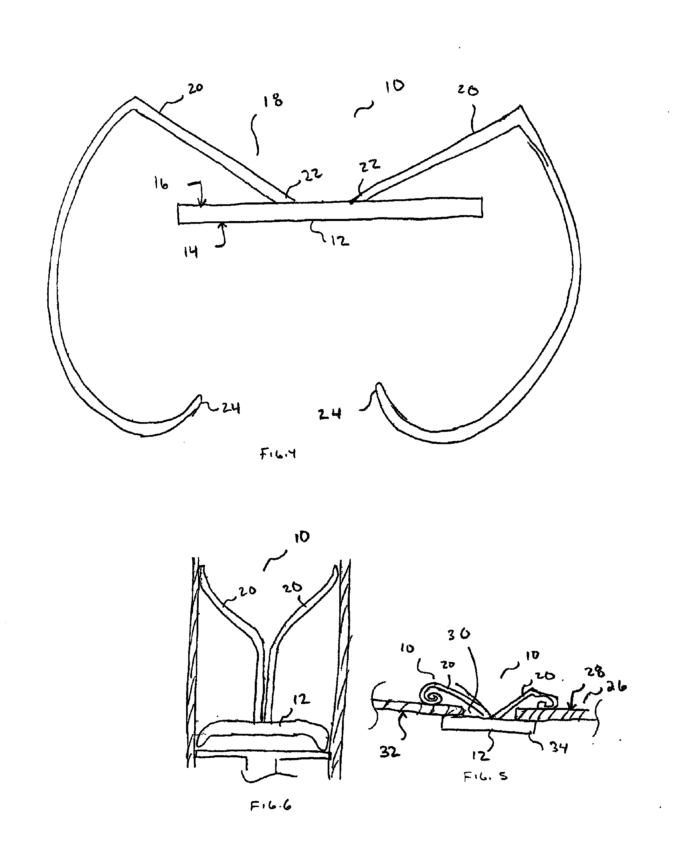

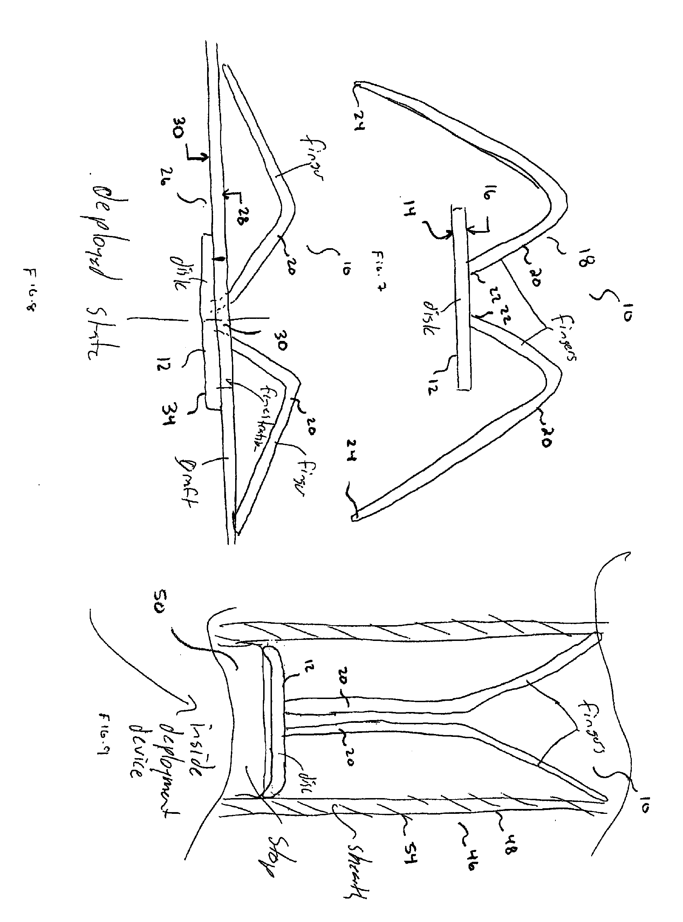

The sealing device 10 may have configurations depending on the intended application. For example, the sealing devices 10 as shown in Figures 4 and 7 respectively, include two biasing members 18 with a more defined angular shape to reduce the overall profile of the sealing device 10 when in the expanded position and when engaged with the stent graft 26 as shown in Figures 5 and 8, respectively.

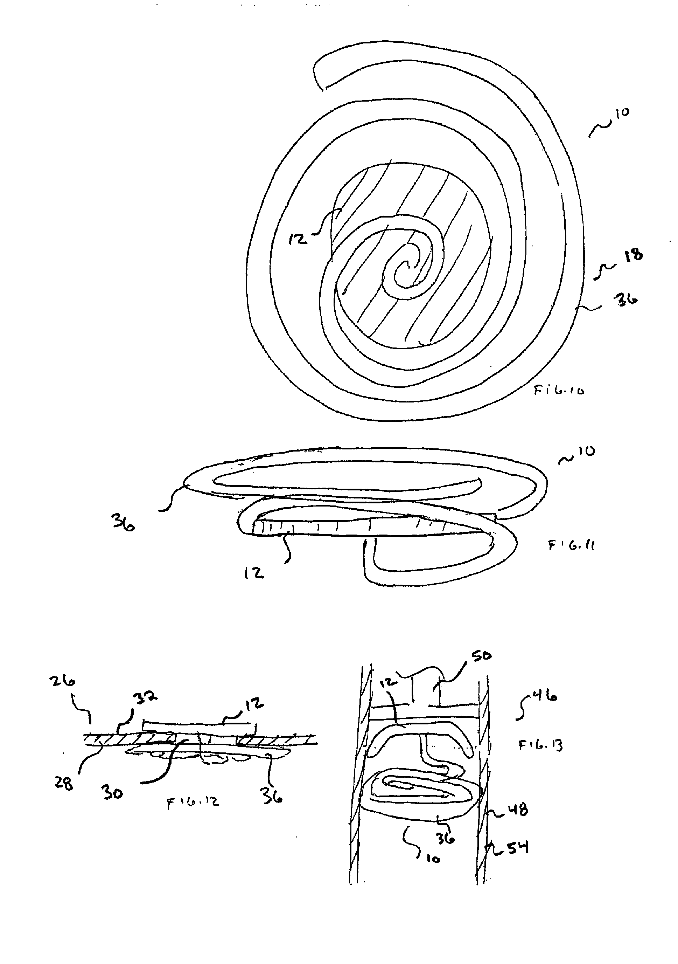

In another embodiment, shown in Figure 10, the biasing member 18 is in the form of a helical-shaped coil 36. The coil 36 has one end attached to the exterior face 14 of the sealing member 12. To provide sufficient biasing force to seal the fenestration 30, the coil 36 must be biased towards the sealing member 12, as shown in Figure 11 when in its natural or relaxed state. This will ensure that the sealing member 12 will be held flush against the fenestration 30 as shown in Figure 12. In this embodiment, the coil 36 is pressed against the outer wall 28 of the stent graft 26, thereby pulling the sealing member 12 against the fenestration. The modifications to the sealing member 12 discussed with respect to the other embodiments also apply here as well.

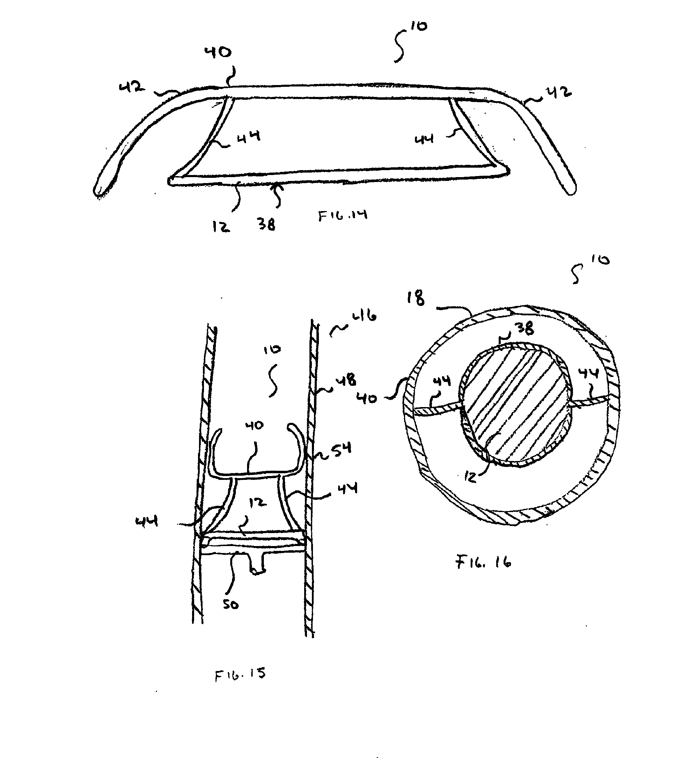

In yet another embodiment, shown in Figures 14 -16, the sealing member 12 is secured to a frame 38, which maintains the shape and rigidity of the sealing member 12 when it is deployed adjacent to the fenestration 30. The increased rigidity of the sealing member 12 as a result of the frame 38 minimizes the amount of overlap required between the sealing member 12 and portion the stent graft 26 surrounding the fenestration 30 to retain the sealing member 12 against the fenestration 30. In the embodiment shown in Figure 14, the biasing member 18 is a circular frame 40 that is configured to be expanded beyond the outer circumference of the fenestration 30 to bias the sealing member 12 against the fenestration 30. As further shown in Figure 14, the circular frame 40 has curved portions 42 that extend beyond the sealing member 12 in the natural or relaxed state. These curved portions 42 can press against the outer wall 28 of the stent graft 26 to pull the sealing member 12 against the inner wall 32 of the stent graft 26 adjacent to the fenestration 30 when disposed through the fenestration 30.

The circular frame 40 may be attached to the frame 38 in a plurality of ways. In this embodiment, the circular frame 40 is attached to the frame 38 with two struts 44. The struts 44 extend from the frame 38 and connect to the circular frame 40 and are located opposite from one another. It can be appreciated that an alternative number of struts 44 may be used and may be oriented differently with respect to each other. In this embodiment, the frame 38, circular frame 40, and struts 44 are made out of the same piece of material, which is preferably made out of a shape memory alloy comprised of Nickel and Titanium, which is commonly referred to as Nitinol, and Teritary alloys, commonly referred to as Nitinol. In other embodiments, the frame members 38, 40 and struts 44 may be made out of different pieces and types of material depending on the particular application.

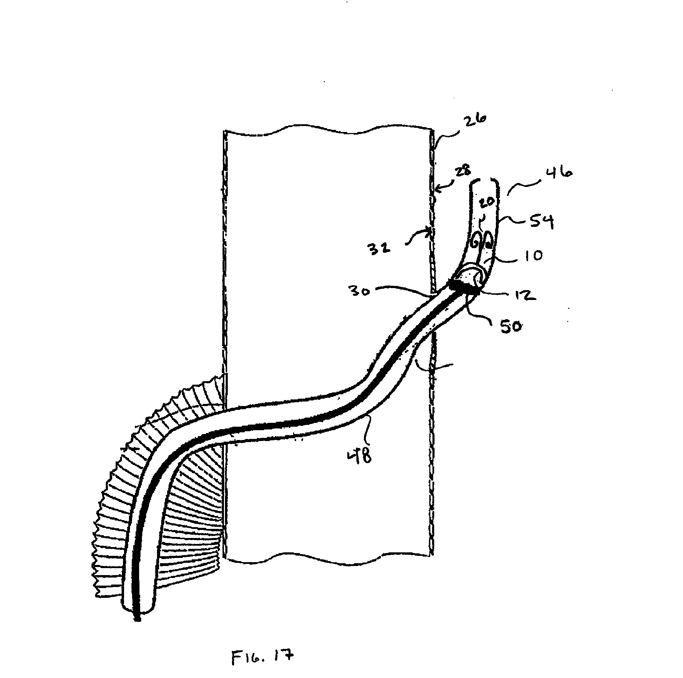

Figures 3, 6, 9, 13, and 15 depict each of the various embodiments of the sealing device 10 disposed within a delivery system 46. The delivery system 46 includes a catheter 48 that is positioned through the fenestration 30 as shown in Figure 17. Positioned within the catheter 48 is a flexible pusher 50 having a disc-shaped end 52 that is used to help place the sealing device 10 in the desired location. As shown in Figure 17, the sealing device 10 is placed within the catheter 48 in a compressed configuration where the sealing member 12 and biasing member 18 are compressed in order to fit into the catheter 48. The compressed configuration of the sealing device 10 minimizes the outer diameter of the catheter 48 that is disposed through the fenestration 30 during the deployment process. The sealing member 12 must have a smaller profile than fenestration 30; otherwise the catheter 48 would not be able to fit through the fenestration 30 to deploy the biasing member 18.

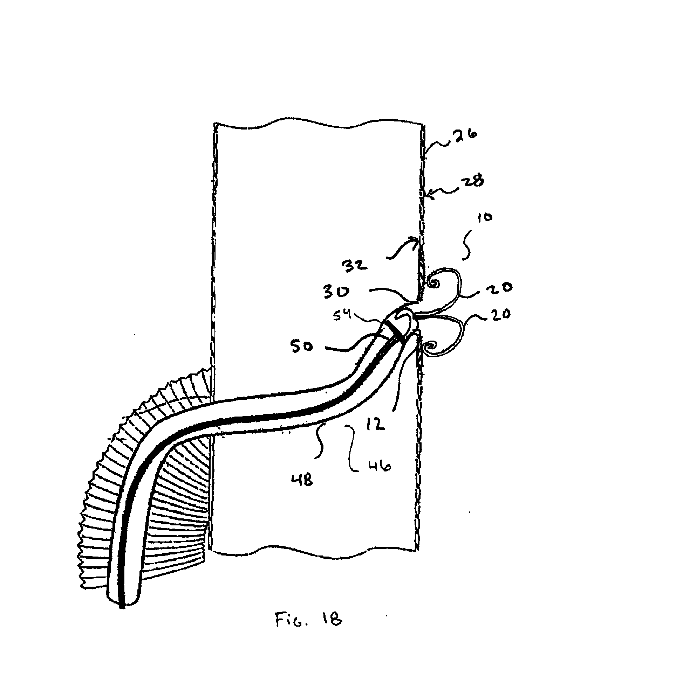

Prior to the deployment, the sealing device 10 is positioned such that the biasing member 18 is located adjacent to the fenestration 30 on the outside of the stent graft 26 and the sealing member 12 is located adjacent to the fenestration 30 but is within the stent graft 26. In other words, the fenestration 30 is located between the sealing member 12 and the biasing member 18. Once the sealing member 12 is positioned at the desired location, the sheath 54 of the catheter is retracted while the sealing device 10 remains positionally fixed. As the sheath 54 is retracted, it releases the biasing member 18, such that it expands to its pre-defmed shape, as shown in Figure 18.

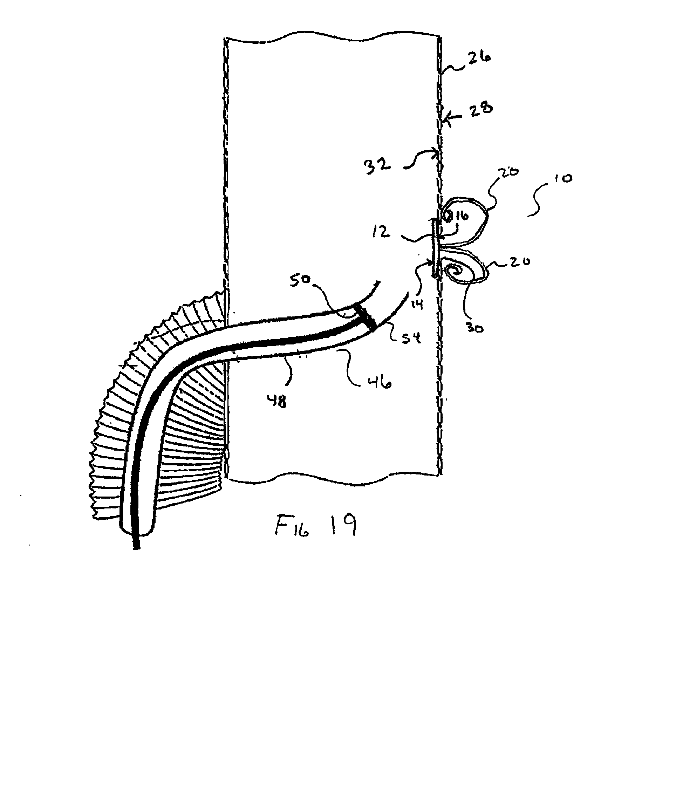

The sheath 54 is further retracted such that it releases the sealing member 12 within the stent graft 26 and adjacent to the fenestration 30. The biasing member 18 pulls the sealing device 12 towards the fenestration 30 thereby sealing the fenestration 30 as shown in Figure 19.

To ensure that the sealing device 10 is properly located with respect to the fenestration 30, the sealing device 10 and/or the delivery system 46 may include radio-opaque markers 56, as shown in Figure 3. The markers 56 enable the sealing device to be properly placed respective to the fenestration 30 and namely ensures that the biasing member 18 is deployed outside of the stent graft and the sealing member 12 is appropriately centered with respect to the fenestration 30 and is deployed within the stent graft 26.

- The delivery system 46 may be deployed through the femoral artery and into the side arm 58 of the stent graft 26. Alternatively, the delivery system 46 may be inserted through one of the legs that is disposed within the iliac artery. It can also be appreciated that the delivery system 46 may be positioned to enter the fenestration 30 from the outside of the stent graft 26 and first deploy the sealing member 12 prior to deploying the biasing member 18. In this scenario, the sealing device 10 will be positioned within the delivery system 46 in the opposite orientation, such that the biasing member 18 would be adjacent to the pusher 50. The remaining steps of deploying the sealing device 10 would be substantially the same as described above. It can be appreciated that depending on the intended use of the sealing device 10, the delivery system 46 may inserted into any other vessel than the ones identified herein.

While the present embodiments have been described in terms of various examples, it will be understood that the embodiments are not limited thereto since modifications may be made to those skilled in the art, particularly in light of the foregoing teachings.

It is to be understood that the features of the various embodiments described herein may be combined with one another as appropriate.

The disclosures in United States patent application no. 61/480,084, from which this application claims priority, and in the abstract accompanying this application are incorporated herein by reference.

## Claims

1. A sealing device for sealing a fenestration within a graft, the sealing device comprising:
a sealing member having an exterior face and an interior face, where the outer diameter of the sealing member is greater than the outer diameter of the fenestration; and
a biasing member extending from the exterior face of the sealing member, the biasing member configured to apply a force against an exterior portion of the graft to cause the sealing member to abut the fenestration.

2. A sealing device according to claim 1, comprising a frame member, the sealing member being disposed within and secured to the frame member, the biasing member extending from the frame member and away from the exterior face of the sealing member.

3. A sealing device according to claim 2, wherein the biasing member and the frame member are formed from the same piece of material.

4. A sealing device according to claim 2 or 3, wherein the biasing member extends from a portion of the frame and is substantially circular in shape, where the outer diameter of the biasing member is greater than the outer diameter of the fenestration.

5. A sealing device according to any preceding claim, wherein the biasing member comprises at least two arcuate fingers extending from the sealing member or from the frame member, each of the at least two fingers configured to apply a force against an exterior portion of the graft.

6. A sealing device according to claim 5, wherein the at least two fingers extend away from the exterior portion of the sealing member.

7. A sealing device according to claim 6, wherein each of the at least two fingers is configured to extend through the fenestration to apply a force against an exterior portion of the graft.

8. A sealing device according to claim 7, wherein at least one of the at least two fingers has a spiral-shaped end or is hooked shaped.

9. A sealing device according to any preceding claim, wherein the biasing member comprises a primary arm having a first end and a second end, where the first end of the primary arm is coupled with the exterior face of the sealing member and the second end comprises at least two or said at least two fingers extending therefrom.

10. A sealing device for sealing a fenestration within a graft, the sealing device comprising:
a sealing member having an exterior face and an interior face, where the outer diameter of the sealing member is greater than the outer diameter of the fenestration; and
a helical biasing member coupled to the sealing member where the helical biasing member is configure to apply a force against an exterior portion of the graft to cause the sealing member to abut the fenestration.

11. A sealing device according to claim 10, wherein the helical biasing device has an outer diameter that is greater than the outer diameter of the fenestration.

12. A sealing device according to claim 10 or 11, comprising a frame that surrounds the sealing member, the frame and helical biasing member being made from the same piece of material.
